Europäisches Patentamt

European Patent Office

Office européen des brevets

(11) Veröffentlichungsnummer: **0 317 735**

**A2**

## (12) EUROPÄISCHE PATENTANMELDUNG

(21) Anmeldenummer: 88115901.6

(22) Anmeldetag: 27.09.88

(51) Int. Cl.4: **C02F 1/32**

(30) Priorität: 25.11.87 DE 3740005

(43) Veröffentlichungstag der Anmeldung:
**31.05.89 Patentblatt 89/22**

(84) Benannte Vertragsstaaten:
**AT BE CH DE ES FR GB GR IT LI LU NL SE**

(71) Anmelder: **Katadyn Produkte AG**
**Industriestrasse 27**
**CH-8304 Wallisellen(CH)**

(72) Erfinder: **Gelzhäuser, Peter, Dipl.-Ing.**
**Schäufeleinstrasse 20**
**D-8000 München 21(DE)**

(74) Vertreter: **Tetzner, Volkmar, Dr.-Ing. Dr. jur.**
**Van-Gogh-Strasse 3**
**D-8000 München 71(DE)**

(54) **Vorrichtung zur Desinfektion von Abwasser.**

(57) Die Erfindung betrifft eine Vorrichtung zur Desinfektion von Abwasser mittels UV-Bestrahlung, enthaltend einen Strömungsraum mit einer UV-Bestrahlungszone, die eine Anzahl von im abwasserdurchströmten Bereich angeordneten UV-Strahlern enthält, wobei der UV-Bestrahlungszone eine Einrichtung zur Erzeugung einer Turbulenz im Abwasser vorgeschaltet und eine Einrichtung zur Erzielung eines gleichmäßigen Wasserstandes nachgeschaltet ist. Eine derartige Vorrichtung gestattet eine zuverlässige Desinfektion großer Abwassermengen.

EP 0 317 735 A2

Fig. 1

## Vorrichtung zur Desinfektion von Abwasser

Die Erfindung betrifft eine Vorrichtung zur Desinfektion von Abwasser mittels UV-Bestrahlung.

Biologisch und chemisch gereinigtes Abwasser weist im allgemeinen noch einen hohen Gehalt an Mikroorganismen auf, der unter anderem dafür verantwortlich ist, daß für natürliche Badegewässer aus seuchenhygienischen Gründen Badeverbote erlassen werden müssen, weil bei Einleitung von Abwasser die natürliche Reinigungskraft des Gewässers nicht ausreicht.

Die Desinfektion des aus einer Kläranlage ablaufenden Abwassers gewinnt daher zunehmend an Bedeutung. Aus Gründen der gewässergüte-wirtschaftlichen Unbedenklichkeit ist dabei der UV-Bestrahlung der Vorzug gegenüber der Behandlung mit Ozon oder mit Chlor zu geben.

Eine Desinfektion von Abwasser mittels UV-Bestrahlung wurde bisher allerdings nur in kleinem Maßstab durchgeführt. Geeignete Anlagen zur Desinfektion großer Abwassermengen mittels UV-Bestrahlung sind bisher nicht bekannt.

Der Erfindung liegt daher die Aufgabe zugrunde, eine Vorrichtung zur Desinfektion von Abwasser mittels UV-Bestrahlung zu schaffen, die einfach aufgebaut ist, einen hohen Zuverlässigkeitsgrad besitzt und die Behandlung großer Abwassermengen gestattet.

Diese Aufgabe wird erfindungsgemäß durch die kennzeichnenden Merkmale des Anspruchs 1 gelöst.

Zweckmäßige Ausgestalltungen der Erfindung sind Gegenstand der Unteransprüche.

Zur Erzielung einer ausreichenden Keimreduktion ist die Kenntnis der Einflußgrößen der UV-Bestrahlung erforderlich. Diese Keimreduktion wird durch das Einwirken der wirksamen UV-Dosis auf den Mikroorganismus erzielt.

Die Art des Mikroorganismus, der physiologische Zustand seiner Zellen sowie die Umgebungsfaktoren bestimmen seine UV-Empfindlichkeit. Zum Abtöten von Bakterien und Viren genügen im allgemeinen geringere Bestrahlungsdosen, als sie für Pilze und Hefen erforderlich sind. Unterschiede im Ernährungszustand und in der Wachstumsphase machen sich ebenso bemerkbar wie unterschiedliche physikalische und chemische Parameter im Bestrahlungsmilieu.

Die Inaktivierung der Mikroorganismen erfolgt durch photochemische Reaktionen mit den bakteriziden UV-C-Strahlen. Die Strahlen von 240 bis 280 nm werden von den Nucleinsäuren absorbiert. Es kommt dabei insbesondere zur Bildung von Dimeren, bevorzugt an den Thyminbasen. Diese Veränderung blockiert sofort die Bindungsmöglichkeit zu den Adeninbasen und macht eine weitere Zellvermehrung und damit den Stoffwechsel unmöglich. Die UV-Desinfektion erfordert daher nur eine kurze Kontaktzeit, wobei das entseuchte Wasser sofort nach Verlassen der UV-Anlage ohne Nachbehandlung ablaufen kann.

Die Bestrahlungsdauer stellt die Expositionszeit der Wasserinhaltsstoffe am Strahler dar und wird durch die Durchflußgeschwindigkeit und die Geometrie in der Strahlungskammer beeinflußt. Dabei muß gewährleistet werden, daß die maximale Durchflußgeschwindigkeit nicht überschritten wird, damit die Bakterien eine genügende UV-Dosis erhalten.

Die Bestrahlungsstärke als zweite Einflußgröße der UV-Bestrahlungsdosis ist der Quotient der auf eine Fläche auftreffenden Strahlungsleistung und dieser Fläche.

Maßgebend für die Bestrahlungsstärke ist die UV-C-Leistung des Strahlers, die z. B. bei Quecksilber-Niederdruckstrahlern ca. 40 % der elektrischen Leistungsaufnahme des Strahlers beträgt. Höhere Stromaufnahmen der Strahler und schwankende Fließgeschwindigkeiten führen schon bei Trinkwasser mit höheren Härtegraden zur Belagsbildung auf den Quarzschutzrohren. Erfindungsgemäß wird daher bei der UV-Bestrahlung von Abwasser besonders darauf geachtet, daß sich die Bestrahlung nicht bis auf einen unwirksamen Wert verringert, weil sich infolge erhöhter Oberflächentemperatur des Quarzschutzrohres die Wasserinhaltsstoffe zu einem undurchdringlichen Belag auf den Quarzschutzrohren niederschlagen und/oder infolge verringerter Transmission des Wassers nicht genügend wirksame UV-Dosis zur Inaktivierung der Mikroorganismen zur Verfügung steht.

Die Strahlerleistung ist abhängig von der Strahlernutzungsdauer. Nach der vom Hersteller garantierten Nutzungsdauer ist die zur Erreichung der Mindestdosis erforderliche Bestrahlungsstärke unterschritten und der Strahler muß ausgetauscht werden.

Die Transmission des Wassers ist definiert als das Verhältnis von durchgelassener Strahlungsleistung zur auffallenden Strahlungsleistung.

Die Annahme, daß die UV-Bestrahlung nur in klarem Wasser wirksam ist, trifft nur teilweise zu. Richtig ist, daß gelöste Stoffe, wie z. B. organische Verbindungen und Salze, eine Abnahme der Bestrahlungsstärke durch Absorption bewirken.

Suspendierte Partikel absorbieren jedoch im Gegensatz zu den gelösten Stoffen die auf die Teilchen auftreffende UV-Strahlung nur zum Teil. Der wesentliche Teil des UV-Lichtes wird von den Partikeln gestreut und geht so für die Keimreduktion nicht verloren. Deshalb werden bei trübem Wasser erheblich bessere Keimreduktionen erzielt, als dies aufgrund der reinen Transmissionsmessung zu erwarten ist.

Unter Berücksichtigung der Einflußgrößen werden bei der erfindungsgemäßen Vorrichtung zur Desinfektion von biologisch gereinigtem Abwasser mittels UV-Bestrahlung die Gesamtkeimzahl und besonders die pathogenen und coliformen Keime reduziert. Die Wirkung der UV-Anlage wird durch eine Filtrationsstufe, die vor der UV-Anlage installiert ist, verbessert. Die guten Ergebnisse der Keimreduktion werden dabei trotz schwankender Suspensa-Gehalte (von 5,5 mg/l bis 16 mg/l) erreicht. Dies resultiert aus der erfindungsgemäßen Strömungsführung, bei der durch die der UV-Bestrahlungszone in Strömungsrichtung vorgeschaltete Einrichtung zur Erzeugung einer Turbulenz im Abwasser eine Zerlegung der Suspensaflocken bewirkt wird und die Bakterien somit ungeschützt der UV-Bestrahlung ausgesetzt werden.

Mit der UV-Bestrahlung werden die Keimzahlen in dem ablaufenden Wasser der Kläranlage so reduziert, daß es sowohl als Brauchwasser für die Reinigung der Klärwerksanlagen verwendet als auch in den Vorfluter eingeleitet werden kann, ohne daß dessen Keimbelastung ansteigt.

Gerade dann, wenn der Vorfluter als Badegewässer ausgewiesen ist, wird die Keimreduktion des Abwassers notwendig, um eine Beibehaltung der Gewässerqualität zu gewährleisten, damit von diesem Badegewässer seuchenhygienisch keine Gefährdung des Badenden ausgeht.

Im Vergleich zu den herkömmlichen chemischen Desinfektionsverfahren hat das UV-Desinfektionsverfahren den Vorteil, daß besonders die Krankheitserreger abgetötet werden, ohne daß dabei neue schädliche Stoffe gebildet werden, wie dies z. B. bei der Desinfektion mit Chlor der Fall ist.

Der Betrieb der erfindungsgemäßen UV-Anlage ist gefahrlos. Besondere Sicherheitsmaßnahmen sind nicht erforderlich. Bei Störungen tritt keine Gefährdung von Personen oder Sachen auf. Besondere Räume für die Aufstellung der Anlage oder die Lagerung der Ersatzstrahler sind nicht erforderlich. Die UV-Anlagen können unmittelbar im Pumpenhaus oder im Ablaufgerinne installiert werden.

Für die erfindungsgemäße Vorrichtung finden Strahlungsquellen Verwendung, die eine hinreichende Ausbeute an UV-Licht gewährleisten.

Ein Ausführungsbeispiel der Erfindung ist in der Zeichnung veranschaulicht. Es zeigen

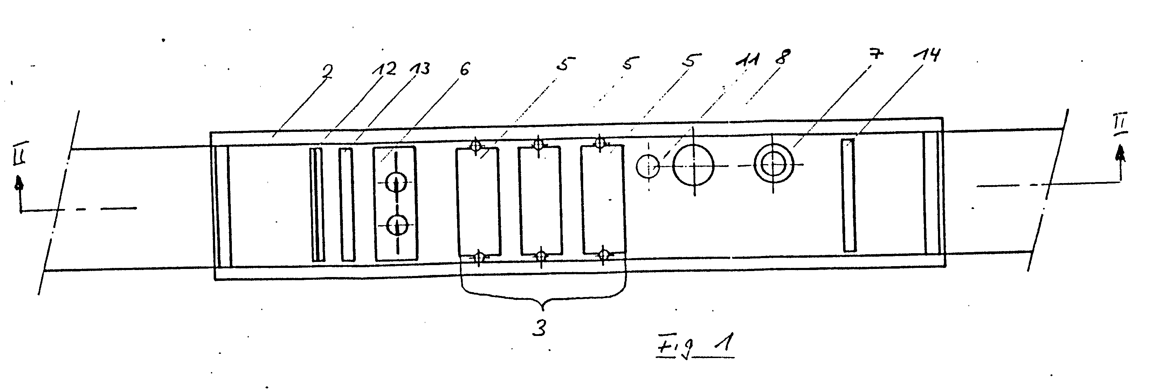

Fig. 1 eine Aufsicht auf ein Ausführungsbeispiel der erfindungsgemäßen Vorrichtung zur Desinfektion von Abwasser mittels UV-Bestrahlung,

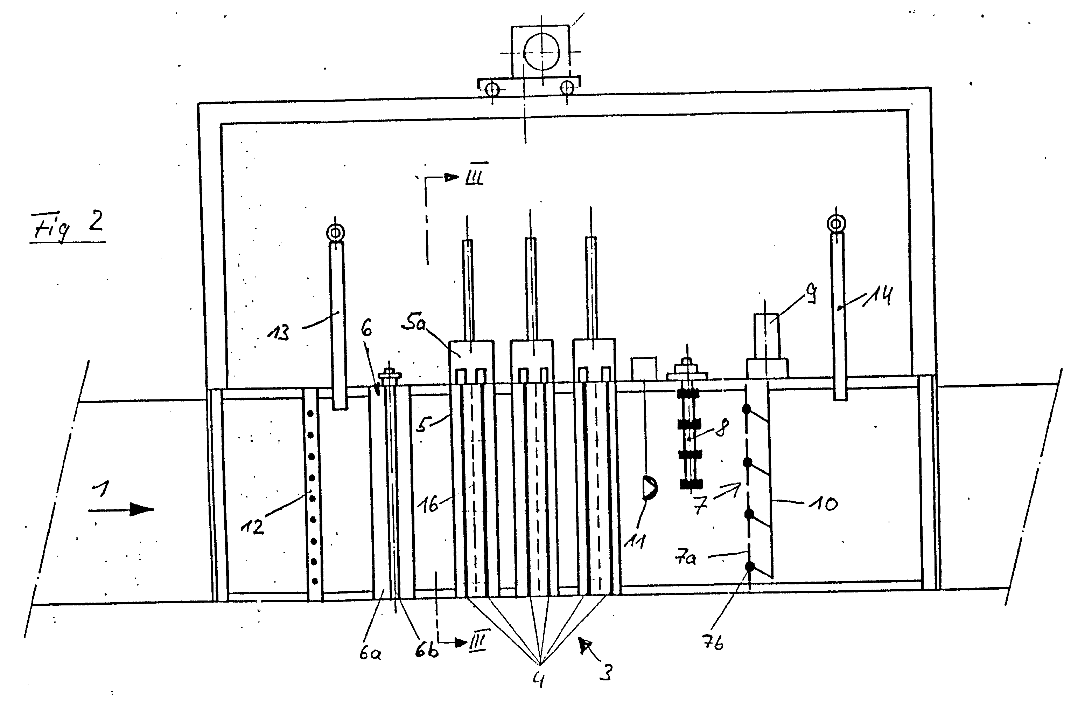

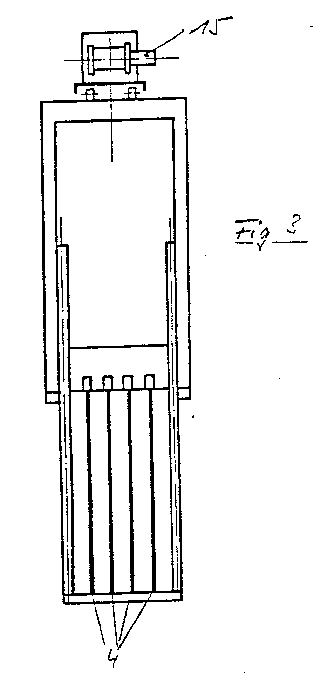

Fig. 2, 3 Schnitte durch die Vorrichtung längs der Linien II-II bzw. III-III.

Die dargestellte Anlage enthält einen von Abwasser (Zuströmrichtung gemäß Pfeil 1) durchsetzten Strömungsraum, der von einer an der Oberseite offenen Wanne 2 gebildet wird. In diesem Strömungsraum befindet sich eine UV-Bestrahlungszone 3, die eine Anzahl von UV-Strahlern 4 enthält, die im abwasserdurchströmten Bereich des Strömungsraumes angeordnet sind.

Die UV-Strahler 4 sind quer zur Strömungsrichtung des Abwassers mit Abstand nebeneinander angeordnet, und zwar beim dargestellten Ausführungsbeispiel vertikal; statt dessen ist jedoch auch eine horizontale Anordnung möglich.

Die UV-Strahler 4 sind dabei in mehreren Reihen - in Strömungsrichtung hintereinander - angeordnet, wobei die Strahler in benachbarten Reihen jeweils auf Lücke versetzt sind. Durch die versetzte Strahleranordnung wird eine turbulente Strömungsführung des drucklosen Gerinnes erreicht, die einen biologischen Bewuchs der Quarzschutzrohre der UV-Strahler auf der Ablaufseite der Strömung weitgehend verhindert und damit lange Wartungsintervalle ermöglicht.

Die UV-Strahler 4 sind gruppenweise zu modulartigen Bausätzen 5 vereinigt, die einzeln eingesetzt und ausgetauscht werden können. Damit kann auf einfache Weise eine Änderung, insbesondere eine Vergrößerung oder Verkleinerung des Strahlerfeldes vorgenommen werden.

Die elektrischen Anschlüsse der einzelnen Strahler sind in einem Klemmenkasten 5a zusammengefaßt, der sich auf den einzelnen Modulen befindet. Der gemeinsame Anschluß des Moduls erfolgt über Steckverbindungen zum Schaltschrank.

Die Steckverbindung muß vor dem Herausziehen des Moduls gelöst werden, so daß die Strahler immer ausgeschaltet sind, wenn sie sich außerhalb des Bestrahlungsraumes befinden.

Der UV-Bestrahlungszone ist in Strömungsrichtung eine Einrichtung zur Erzeugung einer Turbulenz im Abwasser vorgeschaltet. Diese Einrichtung wird durch eine verstellbare Jalousie 6 gebildet, deren Jalousieklappen 6a um quer zur Strömungsrichtung des Abwassers verlaufende Achsen 6b schwenkbar sind, wodurch die Spaltweite zwischen benachbarten Jalousieklappen 6a verändert werden kann. Die Einstellung der Jalousie 6 kann in einfachster Weise von Hand erfolgen.

In Strömungsrichtung ist der UV-Bestrahlungszone eine verstellbare Einrichtung nachgeschaltet, die zur Erzielung eines gleichmäßigen Wasserstandes in der UV-Bestrahlungszone dient. Diese Einrichtung wird durch eine verstellbare Jalousie 7 gebildet, deren Jalousieklappen 7a um quer zur Strömungsrichtung des Abwassers (hier horizontal) verlaufende Achsen 7b schwenkbar sind, wodurch die Spaltweite zwischen benachbarten Jalousieklappen und damit der wirksame Durchlaßquerschnitt veränderbar ist.

Der Füllstand in der UV-Bestrahlungszone wird durch ein Füllstands-Meßgerät 8 überwacht, das einen Stellmotor 9 steuert, der über ein Klappengestänge 10 die Jalousieklappen 7a der Jalousie 7 verstellt.

Durch die vom Füllstandsmeßgerät 8 gesteuerte Jalousie 7 wird erreicht, daß sich der Wasserstand in der UV-Bestrahlungszone immer in der gleichen Höhe befindet, so daß die UV-Strahler 4 ständig in der ganzen wirksamen Länge in das Wasser eintauchen und damit eine optimale Strahlungsverteilung gewährleistet ist.

In der UV-Bestrahlungszone, und zwar beim dargestellten Ausführungsbeispiel auf der Abströmseite hinter der letzten Strahlergruppe, ist eine Fotosonde 11 angeordnet, die die wirksame UV-Strahlung mißt und hierdurch sowohl die Belagbildung an den Quarzschutzrohren der UV-Strahler 4 als auch die Absorption bzw. Trübung des Abwassers überwacht. Diese Fotosonde 11 kann beispielsweise die wirksame UV-Strahlung von 254 nm, aber Trübungen bei anderen Wellenlängen messen. Es können jedoch auch weitere Fotosonden angebracht werden, die entsprechend ihrer Bauart mehr oder weniger sichtbares bzw. unsichtbares Licht aufnehmen und deren Signal zu einem oder mehreren Ausgangssignalen verarbeitet wird.

Zur Ermittlung der wirksamen UV-Dosis ist zweckmäßig ein Mikroprozessor vorgesehen, dem einerseits das Ausgangssignal einer Einrichtung zur Bestimmung des Wasserdurchsatzes und andererseits das Ausgangssignal der die wirksame UV-Strahlung messenden Fotosonde zugeführt wird. Die Einrichtung zur Bestimmung des Wasserdurchsatzes wird durch einen (nicht veranschaulichten)V-förmigen Zulauf gebildet werden, in dem durch Ermittlung der Füllhöhe der Wasserdurchsatz gemessen wird.

Auf der Zulaufseite des von der Wanne 2 gebildeten Strömungsraumes ist ein verstellbarer Rechen 12 angeordnet, durch den Grobteile zurückgehalten werden.

Zur Absperrung des Wasserdurchflusses (für Reinigungszwecke) ist auf der Zulauf- und Ablaufseite des Strömungsraumes je ein Schieber 13 bzw. 14 angeordnet.

Über der Wanne 2 ist eine Laufkatze 15 angeordnet, mit der die einzelnen Anlagenteile, beispielsweise die zu Bausätzen 5 gruppenweise zusammengefaßten UV-Strahler 4, zu Reinigungs-, Kontroll und Reparaturzwecken aus der Wanne 2 nach oben herausgezogen werden können.

Eine Möglichkeit, die UV-Strahler 4 von einer Belagbildung an den Quarzschutzrohren zu reinigen, ohne hierbei den Betrieb der Anlage zu unterbrechen, besteht ferner in der Verwendung einer Wischereinrichtung 16, die nur schematisch (gestrichelt) angedeutet ist. Sie enthält beispielsweise drei Arme, mit denen die ihr zugewandten Flächen von drei UV-Strahlern gleichzeitig gereinigt werden können.

Die jeweils angewandte UV-Dosis wird dem Bedarf angepaßt. Sie wird vor allem durch eine Veränderung der Zahl der UV-Strahler und der Durchflußgeschwindigkeit eingestellt.

Die Fotosonden messen die Bestrahlungsstärke und geben bei Unterschreitung der berechneten UV-Mindestdosis ein Signal ab. Dieses Signal kann weitere Strahlermodule zuschalten, wenn größere Durchflußmengen oder höhere Absorption und/oder Trübung dies erforderlich machen, um die UV-Mindestdosis einzuhalten.

Das Signal kann auch Alarm bei Störung oder den Befehl zur Reinigung während des laufenden Betriebes auslösen.

Ein während des Betriebes möglicher Ausfall einzelner Strahler wird über die Stromaufnahme festgestellt und optisch oder akustisch angezeigt. Der Wechsel einzelner Strahler ist während des laufenden Betriebes ohne Unterbrechung möglich. Ein Betriebsstundenzähler signalisiert das erforderliche Auswechseln der Strahler nach einer bestimmten Zahl von Betriebsstunden (beispielsweise 7000).

Mit diesen Sicherheits- und Überwachungseinrichtungen ist ein vollautomatischer Betrieb der UV-Anlage möglich.

Während bei dem dargestellten Ausführungsbeispiel nur ein einziger Strömungsraum veranschaulicht ist, so versteht es sich doch, daß im Rahmen der Erfindung auch mehrere vom Abwasser durchsetzte Strömungsräume (mit den entsprechenden Einrichtungen) parallelgeschaltet werden können.

## Ansprüche

1. Vorrichtung zur Desinfektion von Abwasser mittels UV-Bestrahlung,
gekennzeichnet durch

a) einen vom Abwasser durchsetzten Strömungsraum (2) mit einer UV-Bestrahlungszone, die eine Anzahl von im abwasserdurchströmten Bereich des Strömungsraumes angeordneten UV-Strahlern (4) enthält,

b) eine der UV-Bestrahlungszone in Strömungsrichtung (1) vorgeschaltete Einrichtung (6) zur Erzeugung einer Turbulenz im Abwasser,

c) eine der UV-Bestrahlungszone in Strömungsrichtung nachgeschaltete verstellbare Einrichtung (7) zur Erzeugung eines gleichmäßigen Wasserstandes in der UV-Bestrahlungszone.

2. Vorrichtung nach Anspruch 1, dadurch gekennzeichnet, daß der Strömungsraum durch eine an der Oberseite offene Wanne (2) gebildet wird, in der die UV-Strahler (4) quer zur Strömungsrichtung des Abwassers mit Abstand nebeneinander angeordnet sind.

3. Vorrichtung nach Anspruch 2, dadurch gekennzeichnet, daß die UV-Strahler (4) in mehreren Reihen - in Strömungsrichtung hintereinander - angeordnet sind, wobei die UV-Strahler in benachbarten Reihen ebenfalls auf Lücke versetzt sind.

4. Vorrichtung nach Anspruch 3, dadurch gekennzeichnet, daß die UV-Strahler (4) zu mehreren, einzeln einsetz- und austauschbaren Gruppen (5) zusammengefaßt sind.

5. Vorrichtung nach Anspruch 1, dadurch gekennzeichnet, daß die Einrichtung zur Erzeugung einer Turbulenz im Abwasser duch eine verstellbare Jalousie (6) gebildet wird, deren Jalousieklappen (6a) unter Veränderung der Spaltweite zwischen den Jalousieklappen um quer zur Strömungsrichtung des Abwassers verlaufende Achsen (6b) schwenkbar sind.

6. Vorrichtung nach Anspruch 1, dadurch gekennzeichnet, daß die Einrichtung zur Erzielung eines gleichmäßigen Wasserstandes in der UV-Bestrahlungszone durch eine verstellbare Jalousie (7) gebildet wird, deren Jalousieklappen (7a) unter Veränderung der Spaltweite zwischen den Jalousieklappen um quer zur Strömungsrichtung des Abwassers verlaufende Achsen (7b) schwenkbar sind, wobei die Verstellung der Jalousie durch ein den Füllstand in der UV-Bestrahlungszone überwachendes Meßgerät (8) steuerbar ist.

7. Vorrichtung nach Anspruch 1, dadurch gekennzeichnet, daß in der UV-Bestrahlungszone, wahlweise auf der An- oder Abströmseite, wenigstens eine die wirksame UV-Strahlung messende und hierdurch die Belagbildung an den Quarzschutzrohren der UV-Strahler (4) sowie Absorption bzw. Trübung des Abwassers überwachende Fotosonde (11) angeordnet ist.

8. Vorrichtung nach Anspruch 7, gekennzeichnet durch einen zur Ermittlung der wirksamen UV-Dosis dienenden Mikroprozessor, dem einerseits das Ausgangssignal einer Einrichtung zur Bestimmung des Wasserdurchsatzes und andererseits das Ausgangssignal der die wirksame UV-Strahlung messenden Fotosonde (11) zugeführt wird.

9. Vorrichtung nach Anspruch 1, dadurch gekennzeichnet, daß auf der Zulaufseite des Strömungsraumes (2) ein verstellbarer Rechen (12) angeordnet ist, durch den Grobteile zurückgehalten werden.

10. Vorrichtung nach Anspruch 1, dadurch gekennzeichnet, daß auf der Zulauf- und Ablaufseite des Strömungsraumes (2) je ein Schieber (13, 14) zur Absperrung des Wasserdurchflusses angeordnet ist.

11. Vorrichtung nach Anspruch 1, dadurch gekennzeichnet, daß in der UV-Bestrahlungszone wenigstens eine zur Abreinigung der UV-Strahler (4) dienende Wischereinrichtung (16) vorgesehen ist.

12. Vorrichtung nach Anspruch 1, dadurch gekennzeichnet, daß über der den Strömungsraum bildenden Wanne (2) eine Laufkatze (15) angeordnet ist, mit der die einzelnen Anlagenteile zu Reinigungs-, Kontroll- und Reparaturzwecken aus der Wanne nach oben herausgezogen werden können.

13. Vorrichtung nach Anspruch 1, dadurch gekennzeichnet, daß mehrere vom Abwasser durchsetzte Strömungsräume parallelgeschaltet sind.

Fig. 1

Fig 2

EP 0 317 735 A2

Fig 3